# EUROPÄISCHE PATENTANMELDUNG

(11) **EP 2 388 581 A1**
(43) Veröffentlichungstag der Anmeldung: **23.11.2011**
(21) Anmeldenummer: 10162947.5
(22) Anmeldetag: 17.05.2010
(51) Int. Cl.: G01N 33/00

(54) **Vorrichtung und Verfahren zur Detektion von Gefahrstoffen mit wenigstens zwei an eine Auswerteeinheit verbundenen austauschbaren Sensorelementen**

(71) Anmelder: Airsense Analytics GmbH, 19061 Schwerin (DE)
(72) Erfinder: Walte, Andreas, Dr., 19059, Schwerin (DE); Münchmeyer, Wolf, 38468, Ehra-Lessien (DE)
(74) Vertreter: Gulde Hengelhaupt Ziebig & Schneider

(57) **Zusammenfassung**

Vorrichtung (10) zur Detektion von Gefahrstoffen mit einer Erfassungseinheit (28) zum Erfassen wenigstens eines Gefahrstoffes und einer Auswerteeinheit (30) zur Bestimmung der Konzentration des wenigstens einen Gefahrstoffes.
Es ist vorgesehen, dass die Erfassungseinheit (28) wenigstens zwei Sensorelemente (34) zur gleichzeitigen Erfassung unterschiedlicher Gefahrstoffe umfasst, und die Sensorelemente (34) austauschbar angeordnet sind und reversibel mit der Auswerteeinheit verbindbar sind.
Ferner ist ein Verfahren zur Detektion von Gefahrstoffen in einem Gasgemisch vorgesehen. Hierzu ist vorgesehen, dass das Gasgemisch auf einen gasförmigen Schadstoff oder zeitgleich mehrere gasförmige Schadstoffe untersucht wird, wobei der gasförmige Schadstoff oder die gasförmigen Schadstoffe durch unterschiedliche Sensormittel erfasst wird bzw. werden und gegebenenfalls die gasförmigen Schadstoffe chemisch so modifiziert werden, dass eine Erfassung mit den zur Verfügung stehenden Sensormitteln erfolgt. Vor der Messung werden Informationen über das Messobjekt herangezogen, um die Detektion und Identifizierung der Schadstoffe zu verbessern.

## Beschreibung

Die Erfindung betrifft eine Vorrichtung und ein Verfahren zur Detektion von Gefahrstoffen, wobei ein Gasgemisch auf das Vorhandensein gasförmiger Schadstoffe untersucht wird.

Es ist bekannt, zur Schädlingsbekämpfung von Gebäuden, auch einzelnen Ausstattungen von Gebäuden, sowie in Lebensmittel verarbeitenden Betrieben, beispielsweise in Lagerräumen, Transportbehältern (Container), Begasungsmittel einzusetzen. Durch den Einsatz dieser Begasungsmittel wird eine effektive Bekämpfung von Ungeziefer möglich, da das Begasungsmittel aufgrund der Gleichverteilung im Raum nahezu überall hingelangt, wo die zu bekämpfenden Ungeziefer sich aufhalten. Nach einer bestimmten Einwirkzeit des Begasungsmittels werden die mit dem Begasungsmittel beaufschlagten Räume und Gegenstände beziehungsweise Produkte entlüftet. Es kann jedoch nicht in jedem Fall sichergestellt werden, dass eine ausreichende Entlüftung erfolgt. Dies kann dazu führen, dass Reste des Begasungsmittels sich noch in den Gebäuden oder in Transportbehältern befinden. Personen, die dann in die Gebäude treten oder die Transportbehälter beispielsweise entleeren wollen oder kontrollieren wollen, wären somit einer Gesundheitsgefährdung ausgesetzt.

Als Begasungsmittel werden beispielsweise schwefelhaltige, chlorhaltige und/oder phosphorhaltige gasförmige Schadstoffe eingesetzt.

Aus US 4,670,405 ist ein tragbares Gerät bekannt, das über eine Anzahl von Sensoren verfügt, mittels denen unterschiedliche gasförmige Schadstoffe in einem Gasgemisch detektiert werden können. Die Sensoren sind hierbei auf zu detektierende Standardschadstoffe ausgelegt. Schadstoffe, die nicht den Standardschadstoffen entsprechen, beziehungsweise Schadstoffe, für deren Detektion keine Sensorelemente zur Verfügung stehen, können mit diesem Gerät somit nicht detektiert werden.

Der Erfindung liegt die Aufgabe zugrunde, ein Verfahren und eine Vorrichtung der gattungsgemäßen Art zu schaffen, mittels denen in einfacher Weise gasförmige Schadstoffe detektiert werden können, insbesondere wenn diese in unterschiedlichen Kombinationen und geringen Konzentrationen auch in Gegenwart anderer Substanzen vorliegen.

Erfindungsgemäß wird diese Aufgabe durch eine Vorrichtung mit den in Anspruch 1 genannten Merkmalen gelöst. Dadurch, dass die Vorrichtung eine Erfassungseinheit zum Erfassen wenigstens eines Gefahrstoffes und eine Auswerteeinheit zur Bestimmung der Konzentration des wenigstens einen Gefahrstoffes umfasst, wobei die Erfassungseinheit wenigstens zwei Sensormittel zur gleichzeitigen Erfassung unterschiedlicher Gefahrstoffe umfasst, und die Sensormittel austauschbar angeordnet sind und reversibel mit der Auswerteeinheit verbindbar sind, ist vorteilhaft möglich, die Vorrichtung in einfacher Weise an unterschiedliche Messsituationen anzupassen. Insbesondere zeichnet sich die Vorrichtung so durch eine hohe Variabilität aus.

In bevorzugter Ausgestaltung der Erfindung ist vorgesehen, dass die Sensormittel durch eine Steckverbindung oder dergleichen austauschbar angeordnet sind. Hierdurch wird ein besonderes einfaches Handhaben möglich. Die Sensormittel selber umfassen vorzugsweise ein Mikrokontrollersystem, über das eine automatische Kalibrierung und Kontaktierung mit der Auswerteeinheit erfolgt. Hierdurch ist in einfacher Weise vor Ort, auch unter robusten Betriebsbedingungen, ein Austausch der Sensoren durch eine Bedienperson ohne weiteres möglich, so dass aufwendige Einstellungen oder Kalibrierungen der gesamten Vorrichtung nicht erforderlich sind.

In weiterer bevorzugter Ausgestaltung der Erfindung ist vorgesehen, dass die Vorrichtung einen Modulator umfasst oder mit einem Modulator zur Zersetzung aufgefundener Gefahrstoffe in messbare chemische Substanzen kombinierbar ist. Hierdurch wird vorteilhaft möglich, gasförmige Schadstoffe, die mit den zur Verfügung stehenden Sensormitteln nicht erfasst werden können, so zu modifizieren, dass als Umwandlungs- und/oder Zersetzungsprodukt ein gasförmiger Stoff entsteht, der mittels der vorhandenen Sensormittel detektierbar ist. Hierdurch ergibt sich eine deutlich vergrößerte Einsetzbarkeit der Vorrichtung. Insbesondere ist es auch so möglich, in einfacher und schneller Weise alle gängigen gasförmigen Schadstoffe zu detektieren, auch solche, für die keine speziellen Sensormittel zur Verfügung stehen. Über diese indirekte Bestimmung der Gefahrstoffe ist ein sehr viel größeres Einsatzspektrum der Vorrichtung möglich.

Darüber hinaus ist in bevorzugter Ausgestaltung der Erfindung vorgesehen, dass als Sensorelemente zur Erfassung gasförmiger Verbindungen Kombinationen verschiedener Gassensoren wie zum Beispiel elektrochemische Zellen und/oder Fotoionisationsdetektoren und/oder NDIR-Sensoren und/oder Metalloxidsensoren und/oder Pellistoren und/oder Ionenmobilitätsspektrometer eingesetzt werden. Um ein tragbares und kleines Gerät zu realisieren, werden bevorzugt kleine Sensoren wie elektrochemische Zellen und/oder Fotoionisationsdetektoren und/oder NDIR-Sensoren und/oder Metalloxidsensoren und/oder Pellistoren verwendet. Zur Detektion radioaktiver Strahlung kann optional ein Gammastrahlendetektor verwendet werden. Hierdurch wird vorteilhaft möglich, die Vorrichtung variabel an konkrete bestehende Messaufgabe anzupassen. Mittels der unterschiedlichen Sensormittel kann zeitgleich das Vorhandensein mehrerer Gefahrstoffe sicher detektiert werden.

Darüber hinaus ist in bevorzugter Ausgestaltung der Erfindung vorgesehen, dass die Vorrichtung eine Kommunikationseinheit zur Kommunikation mit einem Messobjekt und/oder einem Zentralcomputer umfasst, wobei vorzugsweise eine Übertragungsstrecke zwischen der Vorrichtung und dem Zentralcomputer drahtlos über Funk, WLan, Bluetooth oder drahtgebunden über eine USB-Schnittstelle erfolgt. Eine Kommunikation mit dem Messobjekt kann beispielsweise über optische Scanner oder über passive drahtlose Verfahren wie RFID oder aktive Verfahren wie WLan, Bluetooth oder ZigBee erfolgen. Hierdurch wird die Vorrichtung universell einsetzbar und lässt sich auch flexibel auf unübersichtlichem Gelände, beispielsweise bei der Untersuchung und Kontrolle von Transportbehältern (Container) in größeren Hafengeländen und dergleichen, einsetzen.

Die Aufgabe wird ferner durch ein Verfahren mit den in Anspruch 13 genannten Merkmalen gelöst. Dadurch, dass das Gasgemisch auf wenigstens einen Schadstoff untersucht wird, wobei wenigstens ein gasförmiger Schadstoff durch unterschiedliche Sensormittel erfasst wird und gegebenenfalls der gasförmige Schadstoff chemisch so modifiziert wird, dass eine Erfassung mit den zur Verfügung stehenden Sensormitteln erfolgt, wobei vorzugsweise das Gasgemisch zeitgleich auf wenigstens zwei gasförmige Schadstoffe untersucht wird, wobei die wenigstens zwei gasförmigen Schadstoffe durch unterschiedliche Sensormittel erfasst werden und gegebenenfalls die gasförmigen Schadstoffe chemisch so modifiziert werden, dass eine Erfassung mit den zur Verfügung stehenden Sensormitteln erfolgt, wird vorteilhaft möglich, auch komplexe Untersuchungen von Gasgemischen auf mehrere, gegebenenfalls in geringer Konzentration vorhandene gasförmige Schadstoffe mit großer Selektivität durchzuführen. Insbesondere wird möglich, das Gasgemisch aus einem geschlossenen Raum, beispielsweise einem Transportbehälter, über eine Sonde oder dergleichen abzusaugen und der erfindungsgemäßen Vorrichtung zuzuführen. Durch die Modulation können chemische Verbindungen periodisch zersetzt und verschiedenen Sensormitteln, beispielsweise elektrochemischen Sensoren und/oder Fotoionisationsdetektoren, zugeführt werden. Bei der Reaktion eines Schadstoffes kann durch eine Verwendung mehrerer Sensoren das Messergebnis verifiziert werden.

In weiterer bevorzugter Ausgestaltung der Erfindung ist vorgesehen, dass vor der chemischen Analyse des Gasgemisches Informationen über ein zu untersuchendes Messobjekt verwendet werden, um die chemische Analyse auf die zu erwartenden Substanzen abzustimmen. Wenn zum Beispiel ein Container mit einer bestimmten Ware, beispielsweise Schuhe, zu untersuchen ist, kann eine entsprechende Datenbank geladen werden, in der die gasförmigen Schadstoffe gelistet sind, die auch am wahrscheinlichsten auftreten. Hierdurch lässt sich vorteilhaft die Genauigkeit und die Informationsaussage der Messung erhöhen. Insbesondere kann somit die Vorrichtung auf erfahrungsgemäß erwartete oder befürchtete Gefahrstoffe sensibilisiert werden.

Ferner ist in bevorzugter Ausgestaltung der Erfindung vorgesehen, dass die Informationen über das Messobjekt drahtlos übernommen werden, die Analyseergebnisse zwischengespeichert werden und die Informationen und die Analyseergebnisse einem Zentralrechner weitergeleitet werden. Hierdurch wird vorteilhaft eine lückenlose Dokumentationskette gewährleistet, die auch nachweissicher das Erkennen oder Nichterkennen von Gefahrstoffen in zu untersuchenden Messobjekten, beispielsweise Gebäuden oder Transportbehältern, sicherstellt.

Darüber hinaus ist in weiterer bevorzugter Ausgestaltung der Erfindung vorgesehen, dass zur Detektion von Benzol, Toluol, Styrol, Schwefelkohlenstoff, trans-1,2-dichlorethen, Methylbromid und Dibromomethan sowie Phosphin, Ammoniak und Ethylenoxid das Gasgemisch über einen Fotoionisationsdetektor geführt wird, zur Detektion von Blausäure und Phosphin das Gasgemisch über wenigstens eine elektrochemische Zelle geführt wird, zur Detektion von Formaldehyd das Gasgemisch über eine weitere elektrochemische Zelle geführt wird, zur Detektion von Kohlenstoffmonoxid über eine weitere elektrochemische Zelle geführt wird, zur Detektion von gasförmigen Verbindungen, die mit den Sensoren nicht detektiert werden, wie zum Beispiel Sulfuryldifluorid, Chlorpikrin, Carbonylsulfid und Chlormethan, das Gasgemisch über einen Modulator geführt wird, durch den Zersetzungs- beziehungsweise Umwandlungsprodukte generiert werden und die Zersetzungs- beziehungsweise Umwandlungsprodukte über eine weitere elektrochemische Zelle oder weitere elektrochemische Zellen geführt wird. Alternativ kann der Nachweis der gasförmigen Verbindungen, die mit den Sensoren nicht direkt detektiert werden, sondern durch die Wechselwirkung der Zersetzungs- beziehungsweise Umwandlungsprodukte, auch mit den übrigen elektrochemischen Zellen ermöglicht werden. Hierdurch wird vorteilhaft möglich, mittels der Vorrichtung zeitgleich in großem Umfang unterschiedlichste gasförmige Gefahrstoffe zu erkennen und entsprechende Warnsignale zu generieren.

Ferner ist in bevorzugter Ausgestaltung der Erfindung vorgesehen, dass zur Erhöhung der Detektionsfähigkeit eine Antwort aller Sensormittel der Vorrichtung bei nicht aktiviertem Modulator mit der Antwort aller Sensormittel bei aktiviertem Modulator mit Ergebnissen vorheriger Messungen verglichen wird und mit Hilfe von mathematischen Verfahren, beispielsweise Mustererkennungsverfahren, bei positiver Identifizierung ein Alarmsignal ausgegeben wird. Hierdurch ist eine Verifizierung der Messung unmittelbar vor Ort, das heißt am Messobjekt, möglich. Dies erhöht die Genauigkeit der Messergebnisse.

Ferner ist in weiterer bevorzugter Ausgestaltung der Erfindung vorgesehen, dass zur Vermeidung von Bedienfehlern eine Bedienerführung durch die Vorrichtung vor dem eigentlichen Messvorgang auf die einzelnen Schritte im Messablauf hingewiesen wird. Dies kann beispielsweise durch Anzeige in einem Display oder durch eine akustische Wiedergabe erfolgen und sich auf das nacheinander folgende Einlesen von spezifischen Informationen zum Messobjekt beziehungsweise dessen Inhalt, zum Einlesen von Daten der Bedienperson, zum Einführen einer Sonde in das Messobjekt, über die ermittelten Ergebnisse der Messung und die notwendigen Schritte, die einzuleiten sind, um über die Zusammenführung der Informationen der Messergebnisse und die Erstellung von vordefinierten Protokollen in Zusammenhang stehen. Hierdurch wird eine sehr genaue Messung mit hohem Informationsgehalt sichergestellt.

Schließlich ist in weiterer bevorzugter Ausgestaltung der Erfindung vorgesehen, die Bedienung und Handhabung einhändig zu gestalten, indem die Messlanze und die Vorrichtung eine Einheit bilden oder die Messlanze mit der Vorrichtung über einen Schlauch verbunden sind und die Vorrichtung am Körper getragen wird.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figur 1: schematisch den Einsatz einer erfindungsgemäßen Vorrichtung;
- Figur 2: ein Blockschaltbild der erfindungsgemäßen Vorrichtung in einem ersten Ausführungsbeispiel und
- Figur 3: ein Blockschaltbild der erfindungsgemäßen Vorrichtung in einem zweiten Ausführungsbeispiel.

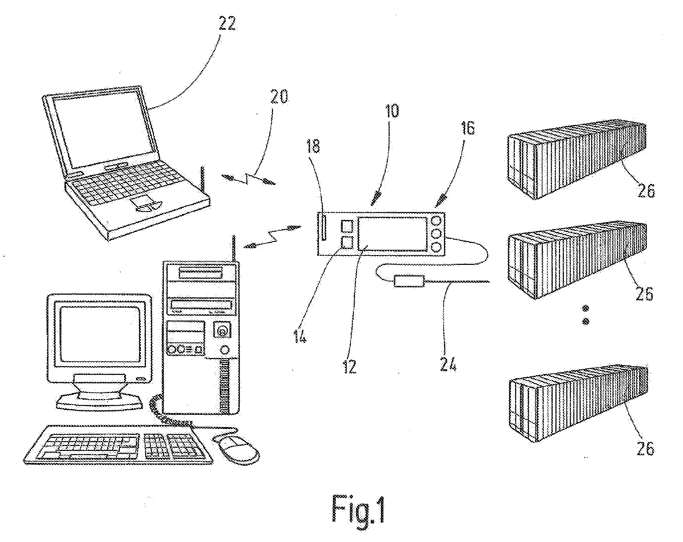

Figur 1 zeigt eine schematische Gesamtübersicht für die Verwendung einer erfindungsgemäßen Vorrichtung 10. Die Vorrichtung 10 ist zur Detektion von Gefahrstoffen geeignet und ist als tragbares Handgerät ausgebildet. Die Vorrichtung 10 umfasst ein Display 12 sowie Betätigungsmittel 14, mittels denen eine Bedienung beziehungsweise Menüführung für die Vorrichtung 10 möglich ist. Die Vorrichtung 10 umfasst ferner eine Anzeigeeinrichtung 16, mittels der ein Messstatus anzeigbar ist. Ferner ist eine Schnittstelle 18 vorgesehen, mittels der eine Übertragungsstrecke 20 zu beziehungsweise von einem Computer 22, Laptop oder dergleichen aufbaubar ist. Die Übertragungsstrecke 20 kann über Funk, WLan, Bluetooth, USB-Verbindung oder dergleichen erfolgen.

Die Vorrichtung 10 umfasst ferner eine Messlanze 24, die mit einem Innenraum hier angedeuteter Transportbehälter 26 in Verbindung zu bringen ist. Die Messlanze 24 kann hierzu beispielsweise durch Dichtungen des Transportbehälters 26 geführt werden, so dass eine Verbindung zwischen dem Innenraum der Transportbehälter 26 und der Umgebung nur durch die Messlanze 24 erfolgt, so dass der Bediener nicht der Luft im Innenraum des Transportbehälters 26 ausgesetzt ist.

Die Vorrichtung 10 umfasst ferner eine hier nicht näher dargestellte Spannungsversorgung, beispielsweise einen wieder aufladbaren Akkumulator.

Die Vorrichtung 10 umfasst ferner ― später noch näher erläutert ― verschiedene Sensormittel zur Detektion unterschiedlicher gasförmiger Schadstoffe, über die eine Detektion der Zusammensetzung des Gasgemisches innerhalb der Transportbehälter 26 möglich ist. Es kann also mit der Vorrichtung 10 festgestellt werden, ob innerhalb der Transportbehälter 26 ein oder mehrere gasförmige Schadstoffe vorhanden sind, die durch ein Öffnen und Betreten des Transportbehälters 26 zu einer Gefährdung von Personen und Umwelt führen würden.

Die in Figur 1 dargestellte Vorrichtung 10 zeigt folgende allgemeine Funktion:
Nach dem Einschalten der Vorrichtung 10 wird der Benutzer nach seiner Identifikation gefragt und aufgefordert, diese einzugeben. Dies kann über eine entsprechende Menüführung über die Betätigungselemente 14 erfolgen. Anschließend werden der Vorrichtung 10 Informationen über die bevorstehenden Messungen eingegeben. Dies können beispielsweise Codes der zu messenden Transportbehälter 26, Angabe zu den in den Transportbehältern 26 enthaltenen Waren, Herkunftsort beziehungsweise Zielort der Transportbehälter 26 und dergleichen sein. Zur Vereinfachung der Eingabe dieser Informationen kann die Vorrichtung 10 mit einem Barcodescanner ausgerüstet sein, der entsprechende an dem Transportbehälter 26 angeordnete Barcodes auslesen kann. Denkbar ist auch, die Vorrichtung 10 mit einer digitalen Kamera auszustatten oder die Vorrichtung 10 über eine Datenverbindung (USB, WLan, Bluetooth oder dergleichen) mit einer digitalen Kamera zu verbinden, um die Transportbehälter 26 dokumentieren zu können. Denkbar ist auch, die Vorrichtung mit einem Mikrofon auszustatten, um somit eine sprachgesteuerte Menüführung zu realisieren. Der Vorrichtung 10 stehen somit alle relevanten Ausgangsinformationen für die durchzuführende Messung zur Verfügung. Diese Ausgangsinformationen werden in einem Speicher der Vorrichtung 10 abgelegt. Dieser Speicher kann ein fest installierter Speicher oder auch ein entnehmbares Speichermittel (USB-Stick, SD-Karte oder dergleichen) sein.

Die Vorrichtung 10 ist anschließend betriebsbereit, wobei die Messung durch einen entsprechenden Startbefehl beispielsweise menügeführt oder durch eine Starttaste begonnen werden kann. Die Messlanze 24 wird in den Innenraum eines ersten Transportbehälters 26 eingebracht. Über eine akustische Meldung oder eine entsprechende Information auf dem Display 12 kann der Beginn der Messung der Bedienperson angezeigt werden. Desgleichen kann über eine weitere akustische Signalisierung oder eine geänderte Anzeige auf dem Display 12 das Ende der Messung angezeigt werden. Die Vorrichtung 10 führt dann selbstständig - wie später noch erläutert wird ― die Untersuchung innerhalb des Transportbehälters 26 auf gasförmige Schadstoffe durch. Die entsprechenden Messdaten werden in der Vorrichtung 10 auf dem bereits erläuterten Speichermittel zwischengespeichert und den entsprechenden Ausgangsinformationen der Messungen, wie Code des Transportbehälters 26 und so weiter, zugeordnet.

Durch die Vorrichtung 10 erfolgt dann eine erste Auswertung der ermittelten Messdaten, damit der Benutzer schnell eine qualitative Aussage erhält. Ob der Transportbehälter 26 ein oder gar mehrere gasförmige Schadstoffe enthält, wird über die Anzeigeeinrichtung 16 optisch mitgeteilt. Die Anzeigeeinrichtung 16 kann hierzu wenigstens eine Leuchtdiode aufweisen, die entweder über eine unterschiedliche Blinkfrequenz oder eine unterschiedliche Farbe dem Benutzer eine qualitative Aussage signalisiert. So kann beispielsweise durch eine grüne Anzeige dargestellt werden, dass die Auswertung der Messung ergeben hat, dass kein Alarmwert erreicht beziehungsweise überschritten wurde. Alle Messwerte liegen deutlich unterhalb der Alarmwerte. Durch eine gelbe Anzeige kann signalisiert werden, dass die Auswertung der Messung ergeben hat, dass zu einigen überprüften Stoffen eingestellte Alarmwerte erreicht aber nicht überschritten wurden. Schließlich kann durch eine rote Signalisierung mitgeteilt werden, dass die Auswertung der Messungen ergeben hat, dass mindestens einer der zu detektierenden Stoffe einen maximale Arbeitsplatzgrenzwert (AGW-Wert, entspricht dem früheren MAK-Wert) überschritten hat. Die entsprechenden Schwellwerte, zu denen Alarm ausgelöst wird oder gerade auch nicht ausgelöst wird, können bei immer wiederkehrenden Messungen bestimmter Stoffe in die Vorrichtung 10 fest einprogrammiert sein. Möglich ist auch eine Anpassung einzelner Werte für bestimmte zu detektierende Stoffe über eine Menüführung an der Vorrichtung 10.

Bei Abschluss der Messung an einem Transportbehälter oder nach Abschluss einer Messserie an mehreren Transportbehältern 26 können die entsprechenden Messergebnisse über die Übertragungsstrecke 20 einem Computer oder dergleichen übertragen werden, wo eine entsprechende Archivierung in einer Datenbank erfolgt. Ferner kann dann eine detailliertere Analyse der ermittelten Messergebnisse, beispielsweise durch Darstellung und Auswertung von Messspektren und dergleichen, erfolgen. Anschließend werden die den einzelnen Transportbehältern 26 zugeordneten Informationen und Messergebnisse dokumentiert und archiviert.

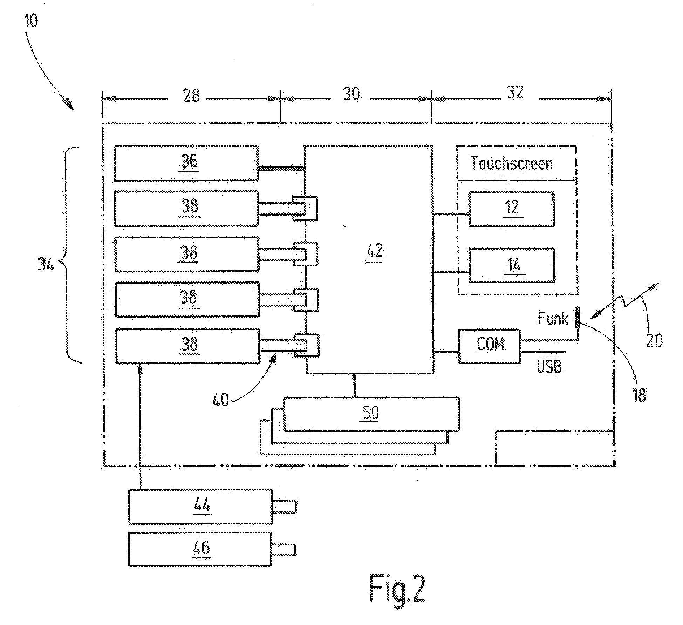

In Figur 2 ist ein Blockschaltbild der Vorrichtung 10 dargestellt. Es wird deutlich, dass die Vorrichtung 10 eine Erfassungseinheit 28, eine Auswerteeinheit 30 und eine Kommunikationseinheit 32 umfasst.

Die Erfassungseinheit 28 besitzt mehrere Sensorelemente 34, von denen eines als Fotoionisationsdetektor 36 ausgebildet ist. Die übrigen Sensorelemente sind beispielsweise als elektrochemische Zellen 38 ausgebildet. Die elektrochemischen Zellen 38 sind über reversibel herstellbare Steckverbindungen 40 mit der Auswerteeinheit 30 verbunden. Durch die Steckverbindungen 40 findet eine Kontaktierung von Mikrokontrollersystemen (zum Beispiel PIC-Controller) der jeweiligen elektrochemischen Zellen 38 mit einem Mikroprozessor oder Mikrocontroller 42 der Auswerteeinheit 30 statt. Die Mikrokontroller der jeweiligen elektrochemischen Zellen 38 ermöglichen eine Speicherung relevanter Daten, wie zum Beispiel die jeweiligen Herstelldaten, Verfalldaten und die Kalibrierparameter. Durch diese Kontaktverbindung zwischen den elektrochemischen Zellen 38 und dem Mikroprozessor 42 findet eine selbsttätige Anpassung beziehungsweise ein Abgleich der elektrochemischen Zellen 38 statt. Durch Ausbildung der Steckverbindungen 40 können die elektrochemischen Zellen 38 ausgetauscht werden. Der Austausch kann beispielsweise erfolgen, um die Vorrichtung 10 mit unterschiedlichen elektrochemischen Zellen 38 zu bestücken, die der gewünschten beziehungsweise erwarteten Messaufgabe entsprechen. So kann flexibel auf entsprechend unterschiedliche Erfassung gasförmiger Schadstoffe mittels der Vorrichtung 10 reagiert werden. Auch ist ein Austausch etwa defekter elektrochemischer Zellen 38 so in einfacher Weise möglich.

In Figur 2 ist hierzu angedeutet, dass die elektrochemischen Zellen 38 beispielsweise auch mit anderen Sensorelementen 44 beziehungsweise 46 austauschbar sind. So kann gegebenenfalls auf unterschiedliche Messaufgaben flexibel reagiert werden.

Mittels des PID-Sensors 36 kann beispielsweise eine Detektion von Lösungsmitteln erfolgen. Durch UV-Strahlung werden alle Moleküle ionisiert, die ein geringeres lonisierungspotenzial als die Energie der UV-Strahlung haben. Mittels einer Spannung werden die erzeugten Ionen an zwei Elektroden nachgewiesen. Hierdurch wird ein Nachweis von relevanten Substanzen, beispielsweise Benzol, Toluol, Styrol, Schwefelkohlenstoff, trans-1,2-dichlorethen, Methylbromid und/oder Dibromomethan sehr gut möglich. Auch können Phosphin, Ammoniak und Ethylenoxid nachgewiesen werden.

Die insgesamt in dem Ausführungsbeispiel vorgesehenen vier elektrochemischen Zellen 38 dienen beispielsweise zur Detektion von Phosgen mit Querempfindlichkeiten zu Phosphin, Arsenwasserstoff, Blausäure, Salzsäure und Schwefelwasserstoff. Eine Nachweisgrenze für Phosphin und Blausäure liegt beispielsweise zwischen 0,1 und 0,5 ppm. Eine weitere elektrochemische Zelle 38 kann zur Detektion von Formaldehyd dienen, mit Querempfindlichkeiten zu Kohlenmonoxid, Ethanol und Methanol. Eine Nachweisgrenze liegt beispielsweise bei 1 ppm. Eine weitere elektrochemische Zelle 38 kann zur Detektion von Schwefeldioxid dienen. Eine Nachweisgrenze liegt beispielsweise bei 1 bis 2 ppm. Schließlich kann eine weitere elektrochemische Zelle 38 zur Detektion von Kohlenstoffmonoxid mit Querempfindlichkeiten zu Ethanol, Blausäure, Wasserstoff und Schwefeldioxid dienen. Hier liegt eine Nachweisgrenze beispielsweise bei 20 ppm.

Der angedeutete Austauschsensor 44 kann beispielsweise zur Detektion von Kohlenstoffdioxid mittels eines NDIR(non-dispersive infrared)-Sensors dienen. Eine Nachweisgrenze liegt beispielsweise bei 1000 ppm. Schließlich ist möglich, das Sensorelement 46 bei Detektion von Gammastrahlung einzusetzen, so dass gegebenenfalls eventuell vorhandene Radioaktivität nachweisbar ist.

Die Austauschsensoren können beispielsweise auch von Metalloxidsensoren und/oder IR-Adsorptionszellen und/oder Pellistoren gebildet sein, oder in weiteren Ausführungsbeispielen auch komplexerer Natur sein, wie zum Beispiel lonenmobilitätsspektrometer.

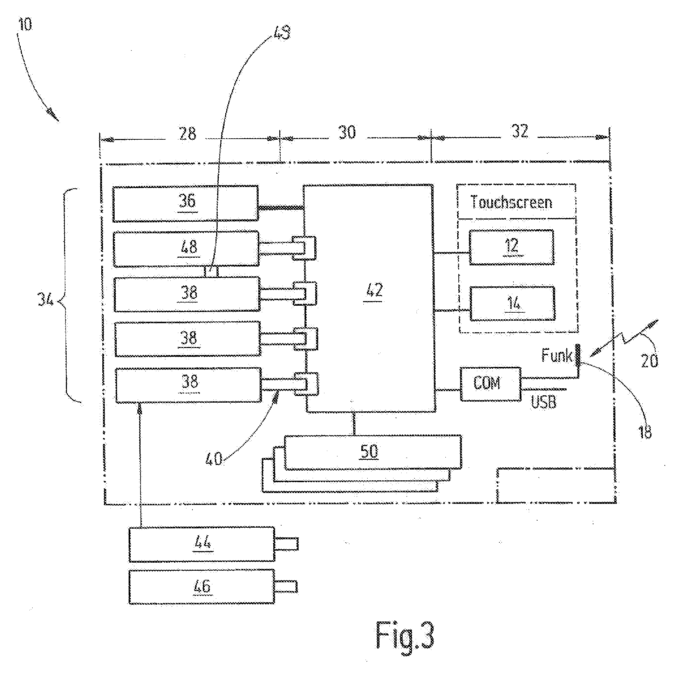

Figur 3 zeigt einen gegenüber dem in Figur 2 dargestellten Ausführungsbeispiel abgewandelten Aufbau der Vorrichtung 10. Gleiche Teile wie in Figur 2 sind mit gleichen Bezugszeichen versehen und nicht nochmals erläutert. Anstelle eines Sensorelementes 38 ist hier ein Modulator 48 vorgesehen, der ebenfalls austauschbar an der Vorrichtung 10 angeordnet ist. Der Modulator 48 ist über eine gasführende Verbindung 49 mit wenigstens einem der Sensorelemente 38 verbunden. Denkbar ist auch, dass der Modulator 48 vor allen oder mehreren Sensorelementen positioniert wird.

Hierdurch wird möglich, dass in dem zu detektieren Gasgemisch vorhandene Bestandteile in Zersetzungs- oder Umwandlungsprodukte durch den Modulator 48 zerlegt werden können, die anschließend dann mit dem Sensorelement 38 detektierbar sind. Dies trifft insbesondere auf solche gasförmigen Schadstoffe zu, die unmittelbar nicht durch die Sensorelemente 38 beziehungsweise 44 und 46 detektierbar sind. Beispielsweise kann so mittels des Modulators 48 Sulfuryldifluorid SO₂F₂ in das Umwandlungsprodukt Schwefeldioxid SO₂ zersetzt werden, das dann mit dem Sensorelement 38, das als elektrochemische Messzelle ausgebildet ist, detektierbar ist.

Sowohl die in Figur 2 als auch in Figur 3 gezeigte Vorrichtung 10 umfassen die Auswerteeinheit 30, der der zentrale Mikroprozessor 42 zugeordnet ist. Über eine Speichererweiterung 50 lassen sich zusätzliche Informationen der Vorrichtung 10 speichern. Die Speichererweiterung 50 kann beispielsweise durch Wechselspeicher, beispielsweise USB-Stick, SD-Cards oder dergleichen, gebildet sein. Somit lassen sich einerseits der Vorrichtung 10 variabel unterschiedliche Messaufgaben vorgeben, die über den zentralen Mikroprozessor 42 den einzelnen Sensorelementen beziehungsweise dem Modulator über die Steckverbindungen 40 zu deren Mikrocontrollern weitergeleitet werden. Andererseits lassen sich mittels der Speichererweiterung 50 die sich aus der Messung ergebenden Daten abspeichern. Durch die austauschbaren Speichermittel kann die Vorrichtung 10 für unterschiedliche Messaufgaben in einfacher Weise umgerüstet werden. Durch die Austauschbarkeit der Sensorelemente 38, 44 46 beziehungsweise des Modulators 48 und der Speichermittel 50 kann somit flexibel innerhalb kürzester Zeit auf sich ändernde Messaufgaben reagiert werden.

Die Vorrichtung umfasst ferner die Kommunikationseinheit 32, der das Display 12 und die Betätigungsmittel 14 zugeordnet sind. Über die Schnittstelle 18 wird die Übertragungsstrecke 20, beispielsweise zu einem Zentralrechner 22 aufgebaut.

### Bezugszeichenliste

- 10: Vorrichtung
- 12: Display
- 14: Betätigungsmittel
- 16: Anzeigeeinrichtung
- 18: Schnittstelle
- 20: Übertragungsstrecke
- 22: Computer, Laptop oder dergleichen
- 24: Messlanze
- 26: Transportbehälter
- 28: Erfassungseinheit
- 30: Auswerteeinheit
- 32: Kommunikationseinheit
- 34: Sensorelemente
- 36: Fotoionendetektor / PID-Sensor
- 38: elektrochemische Zellen
- 40: Steckverbindungen
- 42: Mikroprozessor, Mikrocontroller
- 44: Sensorelemente, Austauschsensor
- 46: Sensorelemente
- 48: Modulator
- 49: Verbindung
- 50: Speichererweiterung

## Patentansprüche

1. Vorrichtung (10) zur Detektion von Gefahrstoffen mit einer Erfassungseinheit (28) zum Erfassen wenigstens eines Gefahrstoffes und einer Auswerteeinheit (30) zur Bestimmung der Konzentration des wenigstens einen Gefahrstoffes, **dadurch gekennzeichnet, dass** die Erfassungseinheit (28) wenigstens zwei Sensorelemente (34) zur gleichzeitigen Erfassung unterschiedlicher Gefahrstoffe umfasst, und die Sensorelemente (34) austauschbar angeordnet sind und reversibel mit der Auswerteeinheit verbindbar sind.

2. Vorrichtung nach Anspruch 1, **dadurch gekennzeichnet, dass** die Sensorelemente (34) durch eine Steckverbindung (40) austauschbar angeordnet sind.

3. Vorrichtung nach einem der vorhergehenden Ansprüche, **dadurch gekennzeichnet dass** die Sensorelemente (34) ein Mikrokontrollersystem umfassen, über das eine automatische Anpassung der spezifischen Sensordaten, wie Verfallsdatum und Kalibrierkurve, und Kontaktierung mit der Auswerteeinheit (30) erfolgt.

4. Vorrichtung nach einem der vorhergehenden Ansprüche, **dadurch gekennzeichnet, dass** als Sensorelemente (34) elektrochemische Zellen (38) und/oder Fotoionendetektoren (36) und/oder NDIR-Sensoren (44) und/oder Radioaktivitätssensoren (46) verwendet werden.

5. Vorrichtung nach Anspruch 4, **dadurch gekennzeichnet, dass** als Sensorelement (34) neben den elektrochemische Zellen (38) und/oder Fotoionendetektoren (36) und/oder NDIR-Sensoren (44) und/oder Radioaktivitätssensoren (46) ein lonenmobilitätsspektrometer verwendet wird.

6. Vorrichtung nach einem der vorhergehenden Ansprüche, **dadurch gekennzeichnet, dass** die Vorrichtung einen Modulator (48) umfasst oder mit einem Modulator (48) kombinierbar ist, zur Zersetzung aufgefundener Gefahrstoffe in messbare chemische Substanzen.

7. Vorrichtung nach einem der vorhergehenden Ansprüche, **dadurch gekennzeichnet, dass** eine Kommunikationseinheit (32) zur Kommunikation mit einem Messobjekt (26) und/oder einem Zentralcomputer (22) vorgesehen ist.

8. Vorrichtung nach einem der vorhergehenden Ansprüche, **dadurch gekennzeichnet, dass** eine Übertragungsstrecke (20) zwischen der Vorrichtung (10) und dem Zentralcomputer (22) drahtlos beispielsweise durch Funk, WLan, Bluetooth oder USB erfolgt.

9. Vorrichtung nach einem der vorhergehenden Ansprüche, **dadurch gekennzeichnet, dass** die Vorrichtung (10) einen Barcodescanner oder ein RFID-Lesegerät umfasst.

10. Vorrichtung nach einem der vorhergehenden Ansprüche, **dadurch gekennzeichnet, dass** die Vorrichtung (10) über eine Sprachsteuerung verfügt.

11. Vorrichtung nach einem der vorhergehenden Ansprüche, **dadurch gekennzeichnet, dass** die Vorrichtung (10) mit Gurten versehen ist, so dass die Vorrichtung (10) am Körper getragen wird, und die Messlanze (24) mit einem Schlauch verbunden ist, so dass eine einhändige Bedienung möglich ist.

12. Vorrichtung nach den vorhergehenden Ansprüche 1 bis 10, **dadurch gekennzeichnet, dass** die Vorrichtung (10) mit der Messlanze (24) eine Einheit bildet, so dass eine einhändige Bedienung möglich ist.

13. Verfahren zur Detektion von Gefahrstoffen in einem Gasgemisch, **dadurch gekennzeichnet, dass** das Gasgemisch auf wenigstens einen Schadstoff untersucht wird, wobei wenigstens ein gasförmiger Schadstoff durch unterschiedliche Sensormittel erfasst wird und gegebenenfalls der gasförmige Schadstoff chemisch so modifiziert wird, dass eine Erfassung mit den zur Verfügung stehenden Sensormitteln erfolgt.

14. Verfahren nach Anspruch 13, **dadurch gekennzeichnet, dass** das Gasgemisch zeitgleich auf wenigstens zwei gasförmige Schadstoffe untersucht wird, wobei die wenigstens zwei gasförmigen Schadstoffe durch unterschiedliche Sensormittel erfasst werden und gegebenenfalls die gasförmigen Schadstoffe chemisch so modifiziert werden, dass eine Erfassung mit den zur Verfügung stehenden Sensormitteln erfolgt.

15. Verfahren nach Anspruch 13 oder 14, **dadurch gekennzeichnet, dass** vor der chemischen Analyse des Gasgemisches Informationen über ein zu untersuchendes Messobjekt (26) verwendet werden, um die chemische Analyse auf die zu erwartenden Substanzen abzustimmen.

16. Verfahren nach einem der Ansprüche 13 bis 15, **dadurch gekennzeichnet, dass** die Informationen über das Messobjekt (26) drahtlos übernommen werden, die Analyseergebnisse zwischengespeichert werden und die Informationen und die Analyseergebnisse einem Zentralrechner (22) weitergeleitet werden.

17. Verfahren nach einem der Ansprüche 13 bis 16, **dadurch gekennzeichnet, dass** zur Vermeidung von Bedienfehlern eine Bedienerführung im Display (12) derart realisiert wird, dass vor der eigentlichen Messung auf die einzelnen Schritte im Messablauf hingewiesen wird.

18. Verfahren nach einem der Ansprüche 13 bis 17, **dadurch gekennzeichnet, dass**
- zur Detektion von Benzol, Toluol, Styrol, Schwefelkohlenstoff, trans-1,2-Dichlorethen, Methylbromid und Dibromoethan, sowie mit etwas geringerer Sensitivität Phosphin, Ammoniak und Ethylenoxid die Gase über einen Fotoionisatiansdetektor (36) geführt werden,
- zur Detektion von Blausäure und Phosphin die Gase über eine elektrochemische Zelle (38) mit Empfindlichkeiten für diese Substanzen geführt werden,
- zur Detektion von Formaldehyd die Gase über eine weitere elektrochemische Zelle geführt werden,
- zur Detektion von Sulfuryldifluorid, Chlorpikrin, Carbonylsulfid und Chlormethan die Gase über eine Modulatoreinheit (48), bei der Zersetzungsprodukte generiert werden, geführt werden und das Sulfuryldifluorid über sein Zersetzungsprodukt (SO₂) mit einer dritten elektrochemischen Zelle erfasst wird, während der Nachweis der anderen Verbindungen durch die Wechselwirkung der Zersetzungsprodukte auch mit den anderen elektrochemischen Zellen erfasst werden und
- die Gase über eine weitere elektrochemische Zelle zur Detektion von Kohlenmonoxid geführt werden.

19. Verfahren nach einem der Ansprüche 13 bis 18, **dadurch gekennzeichnet, dass** zur Steigerung der Detektionsfähigkeit die Antwort aller Sensoren bei nicht aktiviertem Modulator (48) mit der Antwort aller Sensoren bei aktiviertem Modulator (48) mit Ergebnissen vorheriger Messungen, die in einer Datenbank gespeichert werden, verglichen wird und mit Hilfe von mathematischen Verfahren bei positiver Identifizierung ein Alarm ausgegeben wird.
